# FASCICULE DE BREVET EUROPEEN

(11) **EP 0 769 293 B1**
(45) Date de publication et mention de la délivrance du brevet: **11.08.1999**
(21) Numéro de dépôt: 96402098.6
(22) Date de dépôt: 01.10.1996
(51) Int. Cl.: A61K 7/48, A61K 31/135, A61K 31/44

(54) **Utilisation d'au moins un agoniste bêta-adrénergique en tant qu'antagoniste de substance P**
Verwendung mindestens eines beta-adrenergen Agonisten als Antagonist der Substanz P
Use of at least a beta-adrenergic agonist as an antagonist of substance P

(30) Priorité: 23.10.1995 FR 9512447
(43) Date de publication de la demande: 23.04.1997
(73) Titulaire: L'OREAL, 75008 Paris (FR)
(72) Inventeur: Breton, Lionel, 78000 Versailles (FR); De Lacharriere, Olivier, 75015 Paris (FR); Leclaire, Jacques, 91300 Massy (FR)
(74) Mandataire: Tezier Herman, Béatrice

(56) Documents cités:
- EP-A- 0 120 165
- EP-A- 0 544 391
- EP-A- 0 612 525
- EP-A- 0 680 749
- US-A- 4 038 417
- CHEMICAL ABSTRACTS, vol. 124, no. 4, 22 Janvier 1996 Columbus, Ohio, US; abstract no. 37409, XP002009203 & JP 07 258 067 A (KAO CORP.)

## Description

La présente invention concerne l'utilisation d'au moins un agoniste β-adrénergique dans une composition cosmétique en tant qu'antagoniste de substance P ou pour la préparation d'une composition pharmaceutique, plus particulièrement dermatologique, destinée à traiter les désordres associés à un excès de synthèse et/ou de libération de substance P.

L'invention concerne également une composition cosmétique ou pharmaceutique, à usage topique, comprenant au moins un agoniste β-adrénergique.

L'invention a enfin trait à un procédé de traitement cosmétique en vue de diminuer la douleur liée à un excès de synthèse et/ou de libération de substance P ou de diminuer l'effet irritant d'un composé, caractérisé par le fait que l'on applique sur la peau, sur les cheveux et/ou sur les muqueuses une composition comprenant un agoniste β-adrénergique.

Il existe chez les mammifères des polypeptides appartenant à la famille des tachykinines qui induisent sur les fibres musculaires lisses des contractions rapides. Parmi les composés de cette famille, on peut citer la neurokinine β, la neurokinine α et la substance P.

La substance P est un élément chimique polypeptidique (undécapeptide) élaboré et libéré par une terminaison nerveuse. La localisation de la substance P est spécifique des neurones, tant dans le système nerveux central que dans les organes à la périphérie. Ainsi, de très nombreux organes ou tissus reçoivent des afférences de neurones à substance P, il s'agit notamment des glandes salivaires, de l'estomac, du pancréas, de l'intestin (dans celui-ci, la distribution de la substance P est superposée au plexus nerveux intrinsèque de Meissner et d'Auerbach), du système cardio-vasculaire, de la glande thyroïde, de la peau, de l'iris et des corps ciliaires, de la vessie et bien évidemment des systèmes nerveux central et périphérique.

De par la distribution ubiquitaire de la substance P, de très nombreux désordres sont associés à un excès de synthèse et / ou de libération de substance P.

La substance P intervient notamment dans la transmission de la douleur et dans des maladies du système nerveux central (par exemple l'anxiété, les psychoses, les neuropathies, les troubles neurodégénératifs de type démence sénile d'Alzheimer, démence des sidéens, maladie de Parkinson, syndrome de Down, syndrome de Korsakoff, scléroses multiples, schizophrénie), dans des maladies respiratoires (telles que par exemple les broncho-pneumonie) et inflammatoires (telles que par exemple la polyarthrite rhumatoïde), dans des syndromes allergiques (tels que par exemple l'asthme, les rhinites allergiques, les pharyngites allergiques, l'urticaire, les dermatites eczémateuses), dans des maladies gastro-intestinales (telles que par exemple les ulcères, les colites, la maladie de Crohn), dans des désordres cutanés (tels que par exemple le psoriasis, les maladies prurigineuses, l'herpès, les photodermatoses, les dermatites atopiques, les dermatites de contact, les lichens, les prurigos, les prurits, les érythèmes en particulier solaires, les piqûres d'insectes), dans des fibroses et autres troubles de la maturation des collagènes (tels que par exemple la sclérodermie), dans des troubles cardio-vasculaires, des troubles vasospastiques (tels que par exemple les migraines, la maladie de Reynaud), dans des désordres immunologiques, dans des troubles du tractus urinaire (tels que par exemple l'incontinence, la cystite), dans des maladies rhumatismales dans certaines maladies dermatologiques et dans les affections ophtalmologiques (telles que par exemple les conjonctivites, les uvéites, les prurits oculaires, les douleurs oculaires, les irritations).

L'utilisation d'antagoniste de substance P est l'une des alternatives thérapeutiques efficaces dans toutes les affections citées ci-dessus.

Par antagoniste de substance P, on entend tout composé susceptible d'inhiber partiellement, voire totalement, l'effet biologique de la substance P.
Particulièrement, pour qu'une substance soit reconnue comme un antagoniste de substance P elle doit induire une réponse pharmacologique cohérente (incluant ou non sa fixation au récepteur de la substance P) notamment dans l'un des tests suivants :
- la substance antagoniste doit diminuer l'extravasation du plasma au travers de la paroi vasculaire induite par la capsaïcine ou par une stimulation nerveuse antidromique, ou bien
- la substance antagoniste doit provoquer une inhibition de la contraction des muscles lisses induites par l'administration de substance P.

A ce jour, des antagonistes de substance P sont utilisés pour traiter les désordres indiqués ci-dessus.

La demanderesse vient de découvrir que les agonistes β-adrénergiques répondent aux critères d'antagoniste de substance P.

Ainsi, l'invention concerne l'utilisation d'au moins un agoniste β-adrénergique comme antagoniste de substance P dans une composition cosmétique.

L'invention concerne également l'utilisation d'au moins un agoniste β-adrénergique pour la préparation d'une composition pharmaceutique destinée à traiter les désordres associés à un excès de synthèse et/ou de libération de substance P.
De préférence dans la composition pharmaceutique, l'agoniste β-adrénergique est utilisé comme antagoniste de substance P

De préférence, les compositions de l'invention sont destinées à un usage topique.

La présente invention concerne aussi un procédé de traitement cosmétique, caractérisé par le fait qu'il met en oeuvre une composition cosmétique définie selon l'invention.

Les agonistes β-adrénergiques sont des molécules sympathomimétiques qui reproduisent les effets de la stimulation des nerfs sympathiques.

Les sympathomimétiques agissent sur les fibres musculaires lisses. Le prototype en est l'adrénaline. On distingue des récepteurs de nature différente (α ou β) et des sympathomimétiques plus actifs sur certains récepteurs que sur d'autres. C'est le cas des agonistes β-adrénergiques qui sont, comme leur nom l'indique, spécifiques des récepteurs β-adrénergiques (eux-mêmes subdivisés en 2 sous-types principaux, B₁ et B₂).

Il a été décrit des compositions contenant un agoniste β-adrénergique pour le traitement des excès de graisse sur le corps humain (EP-A-120165) ou pour le traitement du psoriasis (US 4038417) ou encore comme agent utilisable dans des bains pour procurer à la peau une sensation de fraîcheur et au corps une sensation de chaleur (JP-A-07258067).
Cependant, aucun des documents de l'art antérieur ne décrit ni ne suggère l'implication de la substance P dans les affections que les compositions décrites sont destinées à combattre et encore moins une quelconque relation entre la substance P et les agonistes β-adrénergiques. L'utilisation d'agonistes β-adrénergiques comme antagoniste de substance P n'a jamais été décrite.

Parmi les agonistes β-adrénergiques utilisables selon l'invention, on peut citer le salbutamol, l'isoprotérénol, le CGP12177, la nylidrine, le salmétérol, le fenotérol, la terbutaline ou le pirbutérol.

Préférentiellement selon l'invention, on utilise le salbutamol.

La quantité d'agoniste β-adrénergique utilisable selon l'invention est bien entendu fonction de l'effet recherché et de la nature de l'agoniste utilisé.
A titre d'illustration, selon l'invention, l'agoniste peut être utilisé en une quantité pondérale représentant de 10⁻⁸ % à 10 % du poids total de la composition et préférentiellement en une quantité représentant de 10⁻⁵ % à 4 % du poids total de la composition.

On a vu préalablement dans le texte des exemples de désordres cutanés liés à un excès de synthèse et/ou de libération de substance P.

Ainsi, l'invention a plus particulièrement pour objet l'utilisation d'au moins un agoniste β-adrénergique dans une composition cosmétique ou pour la préparation d'une composition pharmaceutique destinée à traiter les désordres cutanés tels que par exemple le psoriasis, les maladies prurigineuses, l'urticaire, les dermatites atopiques, les dermatites de contact, les fibroses, les troubles de la maturation du collagène et d'une façon générale les dermatoses prurigineuses et/ou érythémateuses.

Par ailleurs, dans le domaine des désordres cutanés il est connu que certaines peaux sont plus sensibles que d'autres. A cette sensibilité accrue, sont souvent associés des phénomènes comme des irritations cutanées et/ou des dartres et/ou des érythèmes et/ou des sensations dysesthésiques et/ou des sensations d'échauffement et/ou des prurits de la peau et/ou des muqueuses.

Toutefois, les symptômes des peaux sensibles étaient jusqu'à présent mal caractérisés et le problème de ces peaux était, de ce fait, mal défini. En fait, personne ne connaissait exactement le processus mis en cause dans la sensibilité de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques. On assimilait également les peaux sensibles à des peaux allergiques.

Des tests ont été mis au point pour cerner les peaux sensibles, par exemple des tests à l'acide lactique et au DMSO qui sont connus pour être des substances irritantes : voir par exemple l'article de K. Lammintausta et al., Dermatoses, 1988, 36, pages 45-49 ; et l'article de T. Agner et J. Serup, Clinical and Experimental Dermatology, 1989, 14, pages 214-217.

Du fait de la méconnaissance des caractéristiques des peaux sensibles, il était jusqu'à présent très difficile voire impossible de les traiter. En fait, on les traitait indirectement, par exemple en limitant dans les compositions cosmétiques ou dermatologiques l'emploi de produits à caractère irritant tels que les tensioactifs, les conservateurs ou les parfums ainsi que l'emploi de certains actifs cosmétiques ou dermatologiques.

Après de nombreux tests cliniques, la demanderesse a pu déterminer les symptômes liés aux peaux sensibles. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

La demanderesse a pu montrer en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.

La demanderesse a trouvé que les peaux sensibles pouvaient être scindées en deux grandes formes cliniques, les peaux irritables et/ou réactives et les peaux intolérantes.

Une peau irritable et/ou réactive est une peau qui réagit par un prurit, c'est-à-dire par des démangeaisons ou par des picotements, à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche avec ou sans dartres ou à une peau qui présente un érythème.

Une peau intolérante est une peau qui réagit par des sensations d'échauffement, de tiraillements, de fourmillements et/ou de rougeurs, à différents facteurs tels que l'environnement, les émotions, les aliments et certains produits cosmétiques. En général, ces signes sont associés à une peau hyperséborrhéique ou acnéique avec ou sans dartres et à un érythème.

Les cuirs chevelus "sensibles" ont une sémiologie clinique plus univoque : les sensations de prurit et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à certaines préparation cosmétiques, à l'eau dure à forte concentration en calcaire et/ou aux variations de température.

Pour déterminer si une peau est sensible ou non, la demanderesse a également mis au point un test. En effet, après avoir effectué un grand nombre de tests dans le but de définir une peau sensible, elle a trouvé qu'il existait un lien entre les personnes à peau sensible et celles qui réagissaient à une application topique de capsaïcine.

Le test à la capsaïcine consiste à appliquer sur environ 4 cm² de peau 0,05 ml d'une crème comprenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

Jusqu'à présent, la capsaïcine était utilisée comme médicament, en particulier pour traiter les douleurs du zona. La capsaïcine provoque un relargage des neuropeptides, et en particulier des tachykinines qui proviennent de terminaisons nerveuses de l'épiderme et du derme. La demanderesse a constaté que le schéma physiopathologique commun à tous les états des peaux sensibles était lié à une grande aptitude à libérer des tachykinines et plus particulièrement de la substance P dans la peau. Les manifestations dysesthésiques qui sont provoquées par leur libération sont dites "neurogènes".

Personne n'avait établi un lien entre la substance P et la peau sensible. Les signes cliniques de la peau sensible sont essentiellement subjectifs : picotements, fourmillements, prurits, tiraillements, échauffements, et ils s'associent parfois à des érythèmes. Ces signes sont dus à des facteurs extérieurs aspécifiques. Les symptômes apparaissent essentiellement localisés au visage, au cou et au cuir chevelu, mais peuvent apparaître aussi sur tout le corps.

Ainsi, la demanderesse a découvert que l'une des caractéristiques essentielles des peaux sensibles est liée à la libération de substance P et donc que l'utilisation d'antagonistes de substance P peut permettre d'obtenir un effet préventif et/ou curatif des peaux sensibles.

Pour traiter les peaux sensibles, la demanderesse a donc envisagé d'utiliser des antagonistes de substance P. Elle a en effet constaté de manière surprenante que l'incorporation d'un antagoniste de substance P dans une composition destinée à un usage topique permet d'éviter l'irritation et/ou les sensations dysesthésiques et/ou les prurits de la peau.

L'invention concerne donc plus particulièrement l'utilisation d'au moins un agoniste β-adrénergique dans une composition cosmétique ou pour la préparation d'une composition pharmaceutique, ces compositions étant destinées à traiter les peaux sensibles.

La présente invention a encore plus particulièrement pour objet l'utilisation d'au moins un agoniste β-adrénergique dans une composition cosmétique ou pour la préparation d'une composition pharmaceutique, ces compositions étant destinées à prévenir et/ou à lutter contre les irritations cutanées et/ou les dartres et/ou les érythèmes et/ou les sensations d'échauffement et/ou de dysesthésie et/ou les prurits de la peau et/ou les muqueuses.

L'agoniste β-adrénergique peut être utilisé dans une composition qui doit être ingérée, injectée ou de préférence appliquée sur la peau (sur toute zone cutanée du corps), les cheveux, les ongles ou les muqueuses (buccale, jugale, gingivale, génitale, anale, conjonctive). Selon le mode d'administration, cette composition peut se présenter sous toutes les formes galéniques normalement utilisées.

Pour l'injection, la composition peut se présenter sous forme de lotion aqueuse, de suspension huileuse ou sous forme de sérum. Pour les yeux, elle peut se présenter sous forme de gouttes et pour l'ingestion, elle peut se présenter sous forme de capsules, de granulés de sirops ou de comprimés.

Plus particulièrement, l'agoniste β-adrénergique est utilisé dans un composition à usage topique.

L'invention a également pour objet une composition cosmétique ou pharmaceutique, à usage topique, comprenant au moins un agoniste β-adrénergique.

Pour une application topique sur la peau, la composition peut avoir la forme notamment de solution aqueuse ou suspension huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères.

Les compositions peuvent également consister en des préparations solides constituant des savons ou des pains de nettoyage.

Les compositions peuvent aussi être conditionnées sous forme de composition pour aérosol comprenant également un agent propulseur sous pression.

L'agoniste β-adrénergique utilisé selon l'invention peut aussi être incorporé dans diverses compositions pour soins capillaires, et notamment des shampooings, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, des shampooings antiparasitaires, etc.

Les compositions peuvent aussi être à usage bucco-dentaire, par exemple une pâte dentifrice. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol, l'isopropanol et le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention on peut, entre autres, associer au moins un agoniste β-adrénergique à d'autres agents actifs destinés notamment à la prévention et/ou au traitement des affections cutanées.

Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxy-carboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

Ainsi, selon un mode particulier, l'invention concerne une composition comprenant au moins un agoniste β-adrénergique et au moins un agent choisi parmi les agents antibactériens, antiparasitaires, antifongiques, antiviraux anti-inflammatoires, antiprurigineux, anesthésiques, kératolytiques, anti-radicaux libres, anti-séborrhéiques, antipelliculaires, antiacnéiques et/ou les agents modulant la différenciation et/ou la prolifération et/ou la pigmentation cutanée.

De façon avantageuse, selon l'invention au moins un agoniste β-adrénergique peut être associé à des produits à effet irritant utilisés couramment dans le domaine cosmétique ou pharmaceutique, produits qui sont parfois des actifs cosmétiques ou pharmaceutiques. La présence d'un antagoniste de substance P sous la forme d'au moins un agoniste β-adrénergique dans une composition cosmétique ou pharmaceutique comprenant un produit ayant un effet irritant permet d'atténuer fortement, voire de supprimer cet effet irritant.
Cela permet en outre d'augmenter la quantité d'actif à effet irritant par rapport à la quantité d'actif normalement utilisée, en vue d'une efficacité améliorée.

Ainsi, l'invention concerne également une composition cosmétique ou pharmaceutique, de préférence à usage topique, comprenant dans un milieu cosmétiquement ou pharmaceutiquement acceptable au moins un produit à effet irritant, caractérisée en ce qu'elle contient en outre au moins un agoniste β-adrénergique.

Comme produits à effet irritant on peut citer par exemple les tensioactifs (ioniques ou non-ioniques), les conservateurs, les solvants organiques ou les actifs comme les α-hydroxy-acides (acide citrique, malique, glycolique, tartrique, mandélique, lactique), les β-hydroxy-acides (l'acide salicylique et ses dérivés), les α-céto-acides, les β-céto-acides, les rétinoïdes (rétinol, rétinal, acide rétinoïque), les anthralines (dioxyanthranol), les anthranoïdes, les peroxydes (notamment de benzoyle), le minoxidil, les sels de lithium, les antimétabolites, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les actifs dépigmentants (hydroquinone).

L'emploi d'antagoniste de substance P permet notamment de multiplier de 2 à 10 fois la quantité d'actif à effet irritant par rapport à l'état de la technique, sans ressentir tous les inconforts mentionnés ci-dessus. Ainsi, on peut utiliser les hydroxy-acides jusqu'à 50 % du poids de la composition ou les rétinoïdes jusqu'à 5 %, en diminuant notablement leur caractère irritant.

Dans ces compositions, l'agoniste b-adrénergique peut être utilisé de préférence en une quantité allant de 10⁻⁸ % à 10 % en poids par rapport au poids total de la composition, et en particulier en une quantité allant de 10⁻⁵ % à 4 % en poids par rapport au poids total de la composition.

Bien évidemment, les compositions selon l'invention comprenant dans un milieu cosmétiquement ou pharmaceutiquement acceptable au moins un produit à effet irritant et au moins un agoniste b-adrénergique peuvent se présenter sous toute forme galénique connue, comme en particulier celles décrites précédemment dans le texte.

La présente invention a en outre pour objet un procédé de traitement cosmétique en vue de diminuer l'effet irritant d'un composé, caractérisé par le fait que l'on applique sur la peau, sur les cheveux, et/ou sur les muqueuses, une composition comprenant au moins un agoniste b-adrénergique.

La présente invention a également pour objet un procédé de traitement cosmétique en vue de diminuer la douleur liée à un excès de synthèse et/ou de libération de substance P, caractérisé par le fait que l'on applique sur la peau, sur les cheveux et/ou sur les muqueuses une composition comprenant au moins un les cheveux et/ou sur les muqueuses une composition comprenant au moins un agoniste β-adrénergique.

Ces procédés de traitement cosmétique de l'invention peuvent être mis en oeuvre notamment en appliquant les compositions cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs, application d'une lotion pour cheveux sur cheveux mouillés, de shampooings, ou encore application de dentifrice sur les gencives.

Les exemples suivants illustrent l'invention sans la limiter aucunement. Dans les compositions, les proportions indiquées sont des pourcentages en poids, sauf mention contraire.

### Exemple 1 : Activité pharmacologique d'un agoniste β-adrénergique :

### Test fonctionnel in vivo sur modèle de l'inflammation neurogène :

Un test fonctionnel *in vivo* est réalisé sur un modèle d'inflammation neurogène pour mettre en évidence le caractère antagoniste de substance P de l'un des agonistes β-adrénergique, selon la méthode décrite par Xu X.J. et collaborateurs (Neurosciences, 1991, 42, 731-737).

Le test consiste à provoquer une inflammation neurogène par la stimulation antidromique du nerf saphène chez l'animal anesthésié. Ce nerf innerve les territoires cutanés des pattes postérieures.
La stimulation provoque la libération à partir des terminaisons nerveuses de substance P, responsable en partie de l'inflammation neurogène.
L'inflammation neurogène est quantifiée par la mesure de la perméabilité tissulaire au bleu Evans, un marqueur de l'extravasation tissulaire de l'albumine plasmatique qui a lieu au cours de l'inflammation.
Ce modèle de référence est utilisé pour la recherche in vivo d'antagonistes de la substance P.

Les résultats de cette étude sont résumés dans le tableau ci-après :

Ceux-ci représentent la moyenne des résultats de 8 expériences.

| Produit | A | B | C | D | E | F |
|---|---|---|---|---|---|---|
| Concentration* | 5,98 ± 0,65 | 2,44 ± 0,56 | n.d. | n.d. | 1,37 ± 0,32 | 0,32 ± 0,62 |
| % d'inhibition | 0 % | 59 % | 100 % | 100 % | 77 % | 95 % |
| Statistiques | | p < 0,01 | p < 0,001 | p < 0,001 | p < 0,001 | p < 0,001 |

| | | | | | | |
|---|---|---|---|---|---|---|
| A = Témoin (en l'absence de tout inhibiteur) | | | | | | |
| B = Spantide II : 30 nmoles (Antagonistes de SP de référence) | | | | | | |
| C = Salbutamol : 1 µmole | | | | | | |
| D = Isoprotérénol : 1 µmole | | | | | | |
| E = CGP12177, HCl : 1 µmole | | | | | | |
| F = Nylidrine, HCl : 1 µmole | | | | | | |
| * : exprimées en µg/ml de bleu Evans | | | | | | |
| n.d.: non détecté | | | | | | |

Ces résultats démontrent que quelque soit la structure chimique des différents composés de la famille des agonistes β-adrénergiques, l'ensemble de ces molécules exerce, toujours d'une manière relativement importante, une activité de type antagoniste de substance P.

### Exemple 2 :

Effet dose du Salbutamol dans le modèle de stimulation antidromique présenté dans l'exemple 1.

| Produit | Salbutamol | | | | Spantide II |
|---|---|---|---|---|---|
| Dose | 0,1 nmole | 1 nmole | 10 nmoles | 100 nmoles | 30 nmoles |
| % d'inhibition | 17 % | 65 % | 80 % | 100 % | 67 % |
| Statistiques | n.s. | p < 0,05 | p < 0,01 | p < 0,01 | p < 0,01 |

| | | | | | |
|---|---|---|---|---|---|
| n.s. : non significatif. | | | | | |

Les résultats démontrent que l'activité du Salbutamol sur la libération de substance P est dose-dépendante.
Ils démontrent également une activité très importante, environ 30 fois supérieure à celle de la molécule de référence, le Spantide II.

### Exemple 3 :

Exemples de formulations illustrant l'invention. Ces compositions ont été obtenues par simple mélange des différents composants.

| Composition 1 : Lotion antiprurigineuse : | |
|---|---|
| Salbutamol | 0,10 % |
| Antioxydant | 0,05 % |
| Isopropanol | 40,00 % |
| Conservateur | 0,30 % |
| Eau | qsp 100 % |

| Composition 2 : Gel pour le traitement du psoriasis : | |
|---|---|
| Salbutamol | 0,10 % |
| Hydroxypropylcellulose (Klucel H vendu par la | 1,00 % |
| société Hercules) | |
| Antioxydant | 0,05 % |
| Isopropanol | 40,00 % |
| Conservateur | 0,30 % |
| Eau | qsp 100 % |

| Composition 3 : Crème de soin du visage (émulsion huile dans eau) | |
|---|---|
| Isoprotérénol | 0,50 % |
| Stéarate de glycérol | 2,00 % |
| Polysorbate 60 (Tween 60 vendu par la société | 1,00 % |
| ICI) | |
| Acide stéarique | 1,40 % |
| Triéthanolamine | 0,70 % |
| Carbomer | 0,40 % |
| Fraction liquide du beurre de karité | 12,00 % |
| Perhydrosqualène | 12,00 % |
| Antioxydant | 0,05 % |
| Parfum | 0,50 % |
| Conservateur | 0,30 % |
| Eau | qsp 100 % |

| Composition 4 : Shampooing | |
|---|---|
| Salbutamol | 0,05 % |
| Lauryl éther sulfate de Na (2,2 OE) | 12,00 % |
| Hydroxypropylcellulose (Klucel H vendu par la | 1,00 % |
| société Hercules) | |
| Parfum | 0,50 % |
| Conservateur | 0,30 % |
| Eau | qsp 100 % |

| Composition 5 : Gel anti-douleur | |
|---|---|
| Salbutamol | 0,25 % |
| Hydroxypropylcellulose (Klucel H vendu par la | 1,00 % |
| société Hercules) | |
| Antioxydant | 0,05 % |
| Chlorhydrate de lidocaïne | 2,00 % |
| Isopropanol | 40,00 % |
| Conservateur | 0,30 % |
| Eau | qsp 100 % |

| Composition 6 : Crème de soin de l'érythème solaire (émulsion huile-dans-eau) | |
|---|---|
| Isoprotérénol | 0,001 % |
| Stéarate de glycérol | 2,00 % |
| Polysorbate 60 (Tween 60 vendu par la société ICI) | 1,00 % |
| Acide stéarique | 1,40 % |
| Acide glycyrrhétinique | 2,00 % |
| Triéthanolamine | 0,70 % |
| Carbomer | 0,40 % |
| Fraction liquide du beurre de karité | 12,00 % |
| Huile de tournesol | 10,00 % |
| Antioxydant | 0,05 % |
| Parfum | 0,50 % |
| Conservateur | 0,30 % |
| Eau | qsp 100 % |

| Composition 7 : Emulsion H/E destinée au traitement de la peau du visage Phase grasse : | |
|---|---|
| Huile d'amandes d'abricot (triglycérides d'acides oléique-linoléïque) | 14,50 % |
| Fraction liquide de beurre de karité (Triglycérides d'acide palmitique-stéarique-oléïque-linoléïque) | 7,00 % |
| Parahydroxybenzoate de propyle (conservateur) | 0,10 % |
| Mélange d'alcool gras (Alcool stéarylique, alcool arachidylique, alcool béhénylique) | 1,00 % |
| Mono-stéarate de sorbitane (Span 60 de chez ICI) | 2,50 % |
| Mélange d'éthyl-2 héxanoate de cétylstéaryle, myristate d'isopropyle (huile de Purcellin) | 2,00 % |

| Phase aqueuse : | |
|---|---|
| Conservateurs | 0,50% |
| Sel disodique de l'acide éthylène di-amine tétracétique 2H₂O (complexants) | 0,05 % |
| Neutralisant | 0,50 % |
| Gélifiant | 0,70 % |
| Glycérine | 5,00 % |
| Monostéarate de sorbitane oxyéthyléné (20 OE) (Tween 60 de ICI) (tensioactif) | 2,50 % |
| Acide n-octanyol-5-salicylique | 1,00 % |
| Salbutamol | 0,05 % |
| Eau déminéralisée ou permutée | qsp 100 % |

| Composition 8 : Emulsion H/E destinée au traitement de la peau du visage Phase grasse : | |
|---|---|
| Huile d'amandes d'abricot (triglycérides d'acides oléique-linoléïque) | 14,50 % |
| Fraction liquide de beurre de karité (Triglycérides d'acide palmitique-stéarique-oléïque-linoléïque) | 7,00 % |
| Parahydroxybenzoate de propyle (conservateur) | 0,10 % |
| Mélange d'alcool gras (Alcool stéarylique, alcool arachidylique, alcool béhénylique) | 1,00 % |
| Mono-stéarate de sorbitane (Span 60 de chez ICI) | 2,50 % |
| Mélange d'éthyl-2 héxanoate de cétylstéaryle, myristate d'isopropyle (huile de Purcellin) | 2,00 % |

| Phase aqueuse : | |
|---|---|
| Conservateurs | 0,50 % |
| Sel disodique de l'acide éthylène di-amine tétracétique 2H₂O (complexants) | 0,05 % |
| Neutralisant | 0,50 % |
| Gélifiant | 0,70 % |
| Glycérine | 5,00 % |
| Monostéarate de sorbitane oxyéthyléné (20 OE) (Tween 60 de ICI) (tensioactif) | 2,50 % |
| Acide rétinoïque | 0,025 % |
| Salbutamol | 0,05 % |
| Eau déminéralisée ou permutée | qsp 100 % |

## Revendications

1. Utilisation d'au moins un agoniste β-adrénergique pour la préparation d'une composition destinée à traiter les désordres associées à un excès de synthèse et/ou de libération de substance P, à l'exception du psoriasis et des dermatites atopiques.

2. Utilisation selon la revendication 1, caractérisée en ce que l'agoniste β-adrénergique est utilisé comme antagoniste de substance P.

3. Utilisation selon l'une quelconque des revendications précédentes, caractérisée par le fait que la composition est destinée à traiter des désordres cutanés.

4. Utilisation selon la revendication précédente, caractérisée en ce que la composition est destinée à traiter les maladies prurigineuses, l'urticaire, les dermatites de contact, les fibroses, les troubles de la maturation du collagène et d'une façon générale les dermatoses prurigineuses et/ou érythémateuses.

5. Utilisation selon l'une quelconque des revendications précédentes, caractérisée par le fait que la composition est destinée à traiter les peaux sensibles.

6. Utilisation selon l'une quelconque des revendications précédentes, caractérisée en ce que la composition est destinée à un usage topique.

7. Utilisation selon l'une quelconque des revendications précédentes, caractérisée en ce que la composition est destinée à prévenir et/ou lutter contre les irritations cutanées et/ou les dartres et/ou les érythèmes et/ou les sensations dysesthésiques et/ou les sensations d'échauffement et/ou les prurits de la peau et/ou les muqueuses.

8. Utilisation selon l'une quelconque des revendications précédentes, caractérisée en ce que l'agoniste β-adrénergique est utilisé en une quantité pondérale représentant de 10-8 % à 10 % du poids total de la composition.

9. Utilisation selon la revendication précédente, caractérisée en ce que l'agoniste β-adrénergique est utilisée en une quantité pondérale représentant de 10-5 % à 4 % du poids total de la composition.

10. Utilisation selon l'une quelconque des revendications précédentes, caractérisée en ce que l'agoniste β-adrénergique est choisi parmi le salbutamol, l'isoprotérénol, le CGP12177, la nylidrine, le salmétérol, le fenotérol, la terbutaline ou le pirbutérol.

11. Utilisation selon la revendication précédente, caractérisée en ce que l'agoniste β-adrénergique est le salbutamol.

12. Utilisation d'au moins un agoniste β-adrénergique pour la préparation d'une composition destinée à être appliquée sur la peau, sur les cheveux, et/ou sur les muqueuses en vue de diminuer la douleur liée à un excès de synthèse et/ou de libération de substance P.

13. Utilisation d'au moins un agoniste β-adrénergique pour la préparation d'une composition destinée à être appliquée sur la peau, sur les cheveux, et/ou sur les muqueuses en vue de diminuer l'effet irritant d'un composé.

14. Composition cosmétique ou pharmaceutique comprenant dans un milieu cosmétiquement ou pharmaceutiquement acceptable au moins un produit à effet irritant choisi parmi les solvants organiques ou les actifs choisis parmi les β-hydroxy-acides, les α-céto-acides, les β-céto-acides, les rétinoïdes, les anthralines, les anthranoïdes, les peroxydes, le minoxidil, les sels de lithium, les antimétabolites, les teintures ou colorants capillaires, les solutions alcooliques parfumantes, les agents antitranspirants, les actifs dépilatoires ou de permanentes, les actifs dépigmentants caractérisée en ce qu'elle contient en outre un agoniste β-adrénergique.

15. Composition cosmétique ou pharmaceutique selon la revendication 15, caractérisée par le fait qu'elle est à usage topique.

## Claims

1. Use of at least one β-adrenergic agonist for the preparation of a composition intended for treating disorders associated with an excessive synthesis and/or release of substance P, with the exception of psoriasis and atopic dermatitis.

2. Use according to Claim 1, characterized in that the β-adrenergic agonist is used as a substance p antagonist.

3. Use according to any one of the preceding claims, characterized in that the composition is intended for treating skin disorders.

4. Use according to the preceding claim, characterized in that the composition is intended for treating pruriginous diseases, urticaria, contact dermatites, fibroses, collagen maturation disorders and in general pruriginous and/or erythematous dermatoses.

5. Use according to any one of the preceding claims, characterized in that the composition is intended for treating sensitive skins.

6. Use according to any one of the preceding claims, characterized in that the composition is intended for topical use.

7. Use according to any one of the preceding claims, characterized in that the composition is intended for preventing and/or combating skin irritations and/or dartres and/or erythemas and/or dysaesthetic sensations and/or sensations of overheating and/or pruritus of the skin and/or the mucous membranes.

8. Use according to any one of the preceding claims, characterized in that the β-adrenergic agonist is used in a quantity by weight representing from 10-8% to 10% of the total weight of the composition.

9. Use according to the preceding claim, characterized in that the β-adrenergic agonist is used in a quantity by weight representing from 10-5% to 4% of the total weight of the composition.

10. Use according to any one of the preceding claims, characterized in that the β-adrenergic agonist is chosen from salbutamol, isoproterenol, CGP12177, nylidrin, salmeterol, fenoterol, terbutaline or pirbuterol.

11. Use according to the preceding claim, characterized in that the β-adrenergic agonist is salbutamol.

12. Use of at least one β-adrenergic agonist for the preparation of a composition intended to be applied to the skin, to the hair and/or to the mucous membranes in order to reduce the pain linked to an excessive synthesis and/or release of substance P.

13. Use of at least one β-adrenergic agonist for the preparation of a composition intended to be applied to the skin, to the hair and/or to the mucous membranes in order to reduce the irritant effect of a compound.

14. Cosmetic or pharmaceutical composition comprising, in a cosmetically or pharmaceutically acceptable medium, at least one product with an irritant effect chosen from organic solvents or active agents chosen from β-hydroxy acids, α-keto acids, β-keto acids, retinoids, anthralins, anthranoids, peroxides, minoxidil, lithium salts, antimetabolites, hair dyes or colorants, perfuming alcoholic solutions, antiperspirants, active agents for depilation or for permanent waving, depigmenting active agents, characterized in that it contains, in addition, a β-adrenergic agonist.

15. Cosmetic or pharmaceutical composition according to Claim 15, characterized in that it is for topical use.

## Patentansprüche

1. Verwendung mindestens eines β-adrenergen Agonisten zur Herstellung einer Zusammensetzung, die zur Behandlung von Störungen bestimmt ist, die mit einer überhöhten Synthese und/oder Freisetzung der Substanz P verbunden sind, wobei Psoriasis und atopische Dermatitis ausgenommen ist.

2. Verwendung nach Anspruch 1, dadurch gekennzeichnet, daß der β-adrenerge Agonist als Antagonist der Substanz P verwendet wird.

3. Verwendung nach einem der vorhergehenden Ansprüche, dadurch gekennzeichnet, daß die Zusammensetzung zur Behandlung von Hautstörungen eingesetzt werden soll.

4. Verwendung nach dem vorhergehenden Anspruch, dadurch gekennzeichnet, daß die Zusammensetzung zur Behandlung von pruriginösen Erkrankungen, Urtikaria, Kontaktdermatitis, Fibrosen, Störungen der Kollagenbildung und ganz allgemein pruriginösen und/oder erythematösen Dermatosen bestimmt ist.

5. Verwendung nach einem der vorhergehenden Ansprüche, dadurch gekennzeichnet, daß die Zusammensetzung zur Behandlung von empfindlicher Haut bestimmt ist.

6. Verwendung nach einem der vorhergehenden Ansprüche, dadurch gekennzeichnet, daß die Zusammensetzung zur topischen Anwendung bestimmt ist.

7. Verwendung nach einem der vorhergehenden Ansprüche, dadurch gekennzeichnet, daß die Zusammensetzung zur Vorbeugung und/oder Bekämpfung von Hautreizungen und/oder Flechten und/oder Erythemen und/oder dysästhesischen Empfindungen und/oder Hitzegefühlen und/oder Juckreiz der Haut und/oder der Schleimhäute dienen sollen.

8. Verwendung nach einem der vorhergehenden Ansprüche, dadurch gekennzeichnet, daß der β-adrenerge Agonist in einen Gewichtsanteil von 10⁻⁸ bis 10 % des Gesantgewichts der Zusammensetzung verwendet wird.

9. Verwendung nach dem vorhergehenden Anspruch, dadurch gekennzeichnet, daß der β-adrenerge Agonist in einem Gewichtsanteil von 10⁻⁵ bis 4 % des Gesamtgewichts der Zusammensetzung verwendet wird.

10. Verwendung nach einem der vorhergehenden Ansprüche, dadurch gekennzeichnet, daß der β-adrenerge Agonist unter Salbutanol, Isoproterenol, CGP12177, Nylidrin, Salmeterol, Fenoterol, Terbutalin oder Pirbuterol ausgewählt ist.

11. Verwendung nach dem vorhergehenden Anspruch, dadurch gekennzeichnet, daß der β-adrenerge Agonist das Salbutamol ist.

12. Verwendung mindestens eines β-adrenergen Agonisten zur Herstellung einer Zusammensetzung, die auf die Haut, die Haare und/oder die Schleimhäute aufgetragen werden soll, um die Schmerzen zu mindern, die mit einer überhöhten Synthese und/oder Freisetzung der Substanz P verbunden sind.

13. Verwendung mindestens eines β-adrenergen Agonisten zur Herstellung einer Zusammensetzung, die auf die Haut, die Haare und/oder die Schleimhäute aufgetragen werden soll, um die reizende Wirkung einer Verbindung zu vermindern.

14. Kosmetische oder pharmazeutische Zusammensetzung, die in einem kosmetisch oder pharmazeutisch akzeptablen Medium mindestens ein Produkt mit reizender Wirkung enthält, das unter organischen Lösungsmitteln oder Wirkstoffen, die unter β-Hydroxysäuren, α-Ketosäuren, β-Ketosäuren, Retinoiden, Anthralinen, Anthranoiden, Peroxiden, Minoxidil, Lithiumsalzen, Antimetaboliten, Haarfärbenitteln oder Farbstoffen für das Haar, parfümierenden alkoholischen Lösungen, Antitranspirantien, Wirkstoffen zur Haarentfernung oder für Dauerwellen und depigmentierenden Wirkstoffen ausgewählt ist, dadurch gekennzeichnet, daß sie ferner einen β-adrenergen Agonisten enthält.

15. Kosmetische oder pharmazeutische Zusammensetzung nach Anspruch 14, dadurch gekennzeichnet, daß sie zur topischen Anwendung dient.
